(19) Europäisches Patentamt
European Patent Office
Office européen des brevets

(11) Veröffentlichungsnummer : **0 062 615**
**B1**

(12) # EUROPÄISCHE PATENTSCHRIFT

(45) Veröffentlichungstag der Patentschrift : **28.11.84**

(51) Int. Cl.$^3$ : **C 07 D209/48**, C 07 D307/90, C 07 D307/89, C 08 F 2/46

(21) Anmeldenummer : **82810148.5**

(22) Anmeldetag : **02.04.82**

(54) **Tetrasubstituierte Phthalsäurederivate, Verfahren zu deren Herstellung und Verwendung.**

(30) Priorität : **08.04.81 CH 2364/81**

(43) Veröffentlichungstag der Anmeldung : **13.10.82 Patentblatt 82/41**

(45) Bekanntmachung des Hinweises auf die Patenterteilung : **28.11.84 Patentblatt 84/48**

(84) Benannte Vertragsstaaten : **CH DE FR GB IT LI NL SE**

(56) Entgegenhaltungen :
**GB-A- 1 571 742**
**GB-A- 2 075 975**
**US-A- 3 933 862**

(73) Patentinhaber : **CIBA-GEIGY AG**
**Postfach**
**CH-4002 Basel (CH)**

(72) Erfinder : **Fischer, Walter, Dr.**
**Vogesenstrasse 77**
**CH-4153 Reinach (CH)**
Erfinder : **Zweifel, Hans, Dr.**
**Lothringerstrasse 93**
**CH-4056 Basel (CH)**

**EP 0 062 615 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue tetrasubstituierte Phthalsäurederivate, d. h. Phthalsäureanhydride, -imide oder -isoimide, Verfahren zu deren Herstellung und die Verwendung der neuen tetrasubstituierten Phthalsäurederivate als Sensibilisatoren für lichtvernetzbare Polymere oder als Initiatoren, bevorzugt im Gemisch mit Aminen, für die Photopolymerisation äthylenisch ungesättigter Verbindungen oder für die photochemische Vernetzung von Polyolefinen.

Gegebenenfalls alkylierte oder halogenierte, besonders chlorierte, Thioxanthone gehören zu den bekanntesten und wirksamsten Sensibilisatoren für photoinduzierte Vernetzungsreaktionen. Voraussetzung für eine erfolgreiche derartige Anwendung ist eine gute Veträglichkeit des Sensibilisators im Polymeren, d. h. der Sensibilisator muss bis zu erhöhten Konzentrationen mit dem Polymeren mischbar sein. Ferner müssen die Sensibilisatoren in den bei der Verarbeitung der Polymeren zur Anwendung gelangenden Lösungsmitteln gut löslich sein. Die vorerwähnten Thioxanthone genügen diesen Anforderungen nicht in jeder Hinsicht ; insbesondere entmischen sie sich leicht im Polymeren, wordurch deren Sensibilisatorwirkung stark beeinträchtigt wird.

Es ist auch bekannt, dass man die Photopolymerisation von äthylenisch ungesättigten Verbindungen durch aromatische Ketone vom Typ des Benzophenons, des Anthrachinons, Xanthons und Thioxanthons initiieren kann, Es ist weiterhin aus der US Patentschrift 3.759.807 bekannt, dass die Initiatorwirkung solcher aromatischer Ketone durch den Zusatz von organischen Aminen beschleunigt werden kann. Da diese Amine allein meist keine Initiatorwirkung besitzen, wirken sie in Kombination mit aromatischen Ketonen als Aktivatoren oder Beschleuniger. Technisch ist dies von grosser Wichtigkeit, da die Produktionsgeschwindigkeit von photochemisch gehärteten Überzügen oder Druckfarben in erster Linie von der Polymerisationsgeschwindigkeit der ungesättigten Verbindung abhängt.

Es wurden nun neue tetrasubstituierte Phthalsäurederivate der Formel I

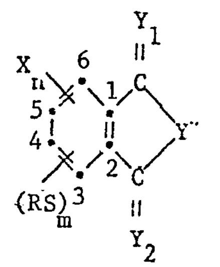

(I)

gefunden, worin

m eine ganze Zahl von 1 bis 4 ist,

n 0, 1, 2, oder 3 darstellt und die Summe von m + n = 4 ist,

$Y_1$ und $Y_2$ Sauerstoff und Y —N(—)—R' oder —O— oder eines von $Y_1$ und $Y_2$ Sauerstoff und das andere =N—R'' und Y —O— bedeuten,

X ein Halogenatom, besonders Chlor oder Brom,

die R (bei $m > 1$) unabhängig voneinander $C_1$-$C_{20}$-Alkyl, $C_{3-5}$-Alkenyl, $C_{3-5}$-Alkinyl, $C_{2-4}$-Monohydroxyalkyl 2,3-Dihydroxy-1-propyl, $—CH_2COOH$, $—CH_2COO—C_{1-4}$-Alkyl, $C_{2-12}$-Halogenalkyl, Benzyl, $C_{5-12}$-Cycloalkyl, Phenyl, Halogenphenyl, Nitrophenyl, Alkyl- oder Alkoxyphenyl mit je 1-4 C-Atomen im Alkyl oder Alkoxy, Monohydroxyphenyl, Monoaminophenyl oder Monoacetylaminophenyl und

R' Wasserstoff, $C_{1-20}$-Alkyl, $C_{2-5}$-Alkenyl, $C_{3-5}$-Alkinyl, $C_{5-10}$-Cycloalkyl, Benzyl, Phenyl, Toluyl, $C_{2-8}$-Monohydroxyalkyl, gesättigter oder ungesättigter aliphatischer $C_{1-4}$-Carbonsäureester von $C_{2-8}$-Monohydroxyalkyl, $C_{1-12}$-Alkyläther von $C_{2-8}$-Monohydroxyalkyl, $—N(C_{1-4}\text{-Alkyl})_2$,

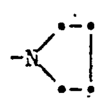 , 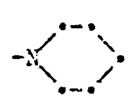 oder 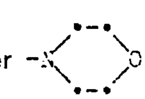

darstellen und

R'' dieselbe Bedeutung wie R' haben kann, aber nicht Wasserstoff darstellt, mit der Massgabe, dass die Gruppen —SR bei M = 1, 2 oder 3 in den Stellungen 4, 4,5 oder 3, 4,5 gebunden sind.

Die Verbindungen der Formel I eignen sich ausgezeichnet zur Verwendung als Sensibilisatoren für lichtvernetzbare Polymere. Sie zeichnen sich insbesondere durch eine gute Verträglichkeit mit dem Polymeren, eine gute Löslichkeit in üblichen organischen Lösungsmitteln und eine hohe Photoempfindlichkeit aus. Überdies kann die UV-Absorption so beeinflusst werden, dass die erfindungsgemässen Verbindungen auch bei Bestrahlung mit langwelligem UV-Licht (bis ca. 450 nm) eine sensibilisierende

Wirkung ausüben und so die Vernetzung der photoempfindlichen Polymeren bewirken. Photosensibilisierende Phthalimide und Phthalsäureanhydride mit Donoren in 3- bis 6-Stellung waren bisher nicht bekannt.

Die Verbindungen der Formel I eignen sich ferner, bevorzugt im Gemisch mit organischen Aminen, als Initiatoren für die Photopolymerisation äthylenisch ungesättigter Verbindungen oder für die photochemische Vernetzung von Polyolefinen.

Definitionsgemässe Alkyl-, Monohydroxyalkyl-, Halogenalkyl-, Alkenyl- und Alkinylgruppen R, R′ oder R″ sowie Alkylsubstituenten in Gruppen R, R′ oder R″ können geradkettig oder verzweigt sein. Halogenalkylgruppen R können durch ein oder mehrere Halogenatome, wie Chlor oder Brom und insbesondere Fluor, substituiert sein. Als Beispiele definitionsgemässer Alkyl-, Monohydroxyalkyl-, $—CH_2COO—C_{1-4}$-Alkyl-, Halogenalkyl-, Alkenyl-, Alkinyl- und $—N(C_{1-4}\text{-Alkyl})_2$-Gruppen sowie definitionsgemässer Ester oder Aether von Monohydroxyalkylgruppen R, R′ oder R″ seien erwähnt: Methyl, Aethyl, n-Propyl, Isopropyl, n-, sek- und tert-Butyl, n-Pentyl, 2- oder 3-Pentyl, n-Hexyl, N-Heptyl, 3-Heptyl, n-Octyl, n-Decyl, n-Dodecyl, n-Tridecyl, n-Tetradecyl, n-Hexadecyl, n-Octadecyl und Eicosyl; 2-Hydroxyäthyl, 2- und 3-Hydroxypropyl, 3- und 4-Hydroxybutyl; $—CH_2COOCH_3$, $—CH_2COOC_2H_5$; 2-Chloräthyl, 2-Bromäthyl, 2,2,2-Trifluoräthyl, $—CH_2CH_2C_4F_9$, $—CH_2CH_2C_6F_{13}$, $—CH_2CH_2C_8F_{17}$ und $—CH_2CH_2C_{10}F_{21}$; Vinyl, Allyl, Methallyl, 2-Butenyl, 4-Pentenyl; 2-Propinyl, 3-Butinyl und 4-Pentinyl; N,N-dimethylamino, N,N-Diäthylamino, N-Methyl-N-äthylamino, N,N-Di-n-propylamino, N,N-Di-n-butylamino; $—CH_2CH_2OCOCH_3$, $—CH_2CH_2CH_2OCOC_2H_5$, $—CH_2CH_2OCO—n \cdot C_4H_9$, $—CH_2CH_2OCOCH{=}CH_2$, $—CH_2CH_2OCOC(CH_3){=}CH_2$, $—CH_2CH_2OCOCH{=}CHCH_3$, $—CH_2CH_2OCH_3$, $—CH_2CH_2CH_2OC_2H_5$, $—CH_2CH_2O—n \cdot C_4H_9$ und $—CH_2CH_2CH_2O—n \cdot C_8H_{17}$.

Alkylgruppen R, R′ und R″ sind bevorzugt geradkettig und weisen 1-12 und insbesondere 1-10 C-Atome auf. Bevorzugte Alkenyl- und Alkinylgruppen R, R′ oder R″ sind: Vinyl (R′ oder R″), Allyl, Methallyl, 2-Butenyl und 2-Propinyl. Halogenalkylgruppen R sind vorzugsweise ebenfalls geradkettig und weisen 1-10 C-Atome auf. Besonders bevorzugt sind $—CH_2CF_3$ und vor allem $—CH_2CH_2C_pF_{2p+1}$ mit p = 6, 8 oder 10.

Beispiele von Cycloalkylgruppen R, R′ oder R″ sind Cyclopentyl, Cyclohexyl, Cyclooctyl und Cyclododecyl. Bevorzugt ist Cyclohexyl. Als Toluylgruppe R′ bzw. R″ kommt vor allem p-Toluyl in Betracht.

Halogenphenyl-, Nitrophenyl-, Alkylphenyl- und Alkoxyphenylgruppen R können ein- oder mehrfach substituiert sein. Bevorzugt sind Phenylgruppen mit einem oder zwei der genannten Substituenten. Als Halogenatome kommen z. B. Fluor, Brom und insbesondere Chlor in Betracht. Beispiele derartiger Gruppen sind: 3-Chlor- oder 3-Bromphenyl, 3,4-Dichlorphenyl, 2,5-Dichlorphenyl, 3,4-Dibromphenyl, 4-Chlor-, 4-Brom- oder 4-Fluorphenyl; 3-oder 4-Nitrophenyl, 3,5-Dinitrophenyl; o- m- und p-Toluyl, 3,4-Dimethylphenyl, 4-Aethylphenyl, 4-n-butylphenyl; 2-, 3- oder 4-Methoxyphenyl, 3- und 4-Aethoxyphenyl, 2,5-Dimethoxyphenyl, 3,4-Diäthoxyphenyl, 4-n-Propoxyphenyl, 4-Isopropoxyphenyl und 4-n-Butoxyphenyl.

Bevorzugt sind Verbindungen der Formel I, worin für Y, $Y_1$, $Y_2$, m und n das unter Formel I Angegebene gilt, X Chlor oder Brom, die R (bei $m > 1$) unabhängig voneinander geradkettiges $C_{1-12}$-Alkyl, 2-Hydroxyäthyl, Phenyl, p-Toluyl, 2-, 3- oder 4-Methoxyphenyl, 4-Chlorphenyl, 2,5-Dichlorphenyl, 4-Nitrophenyl, 4-Acetylaminophenyl, 2- oder 4-Aminophenyl, 4-Hydroxyphenyl oder $—CH_2CH_2C_pF_{2p+1}$ mit p = 6, 8 oder 10 und R′ Wasserstoff, geradkettiges $C_{1-12}$-Alkyl, $C_{5-6}$-Cycloalkyl, Benzyl, Phenyl, Toluyl oder $C_{2-4}$-Monohydroxyalkyl darstellen und R″ dieselbe Bedeutung wie R′ haben kann, jedoch nicht Wasserstoff darstellt. Besonders bevorzugt stellen dabei die R unabhängig voneinander geradkettiges $C_{1-12}$-Alkyl, 2-Hydroxyäthyl, $—CH_2CH_2C_8F_{17}$, Phenyl, p-Toluyl, 2- oder 4-Methoxyphenyl, 4-Chlorphenyl, 2,5-Dichlorphenyl oder 4-Nitrophenyl, R′ Wasserstoff, geradkettiges $C_{1-10}$-Alkyl, Phenyl, p-Toluyl oder 2-Hydroxyäthyl und R″ geradkettiges $C_{1-10}$-alkyl, Phenyl, p-Toluyl oder 2-Hydroxyäthyl dar.

Ganz besonders bevorzugt sind Verbindungen der Formel I, worin für Y, $Y_1$, $Y_2$, m und n das unter Formel I Angegebene gilt, X Chlor oder Brom, die R unabhängig voneinander geradkettiges $C_{1-2}$-Alkyl, vor allem $C_{1-10}$-Alkyl, 2-Hydroxyäthyl, $—CH_2CH_2C_8F_{17}$, Phenyl, p-Toluyl, 2- oder 4-Methoxyphenyl, 4-Chlorphenyl oder 4-Nitrophenyl, R′ Wasserstoff, geradkettiges $C_{1-10}$-Alkyl, besonders $C_{1-4}$-Alkyl, 2-Hydroxyäthyl oder Phenyl und R″ Phenyl darstellen. Bei m = 2 haben die R in den Gruppen —SR bevorzugt je dieselbe Bedeutung. Des weiteren bevorzugt sind Verbindungen der Formel I mit m = 4, wobei die R der in 3,6- und 4,5-Stellung gebundenen Gruppen —SR je paarweise dieselbe Bedeutung haben oder aber alle vier R gleiche Gruppen darstellen.

Die Verbindungen der Formel I können in an sich bekannter Weise hergestellt werden, z. B. dadurch, dass man eine Verbindung der Formel II

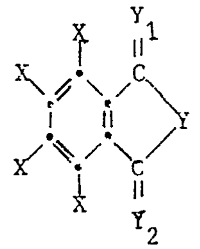

(II)

mit einer oder mehreren Verbindungen der Formel III

RSH (III)

oder Salzen von Verbindungen der Formel III umsetzt, dabei erhaltene Verbindungen der Formel I mit n = 1, 2 oder 3 gegebenenfalls mit weiterer Verbindung der Formel III bzw. weiterem Salz davon umsetzt und gegebenenfalls Verbindungen der Formel I, worin eines von $Y_1$ und $Y_2$ Sauerstoff und das andere =N—R″ und Y —O— bedeuten, zu den entsprechenden Imiden isomerisiert. In den Formeln II und III haben X, Y, $Y_1$, $Y_2$ und R die unter Formel I angegebene Bedeutung. Die Umsetzung mit Verbindungen der Formel III oder Salzen davon zu Verbindungen der Formel I kann demnach direkt oder stufenweise erfolgen. Dabei können auch Gemische verschiedener Verbindungen der Formel I entstehen.

Als Salze von Verbindungen der Formel III kommen sowohl Salze mit anorganischen als auch organischen Basen in Betracht. Bevorzugt sind Alkalimetall- und quaternäre Ammoniumsalze, wie die Na, K-, Tetramethyl-, Tetraäthyl-, Benzyltrimethyl- und Benzyltriäthylammoniumsalze. Die genannten Salze können als solche eingesetzt oder in an sich bekannter Weise in situ gebildet werden. Bevorzugt setzt man die Verbindungen der Formel II in Gegenwart einer Base mit einer Verbindung der Formel III um. Als Basen verwendet man dabei mit Vorteil tertiäre Amine, wie Triäthylamin oder Pyridin ; Alkalimetallacetate, -hydrogencarbonate oder -carbonate, vor allem Triäthylamin und besonders Kaliumcarbonat. Zweckmässig verwendet man 1,0 bis 3 Äquivalente Base, bezogen auf die Verbindung der Formel III.

Die obige Umsetzung kann in wässrigem Medium durchgeführt werden, wird jedoch mit Vorteil in Gegenwart eines inerten organischen Lösungsmittels bei Temperaturen zwischen 0 °C und der Siedetemperatur des Lösungsmittels, bevorzugt bei 0-30 °C, vorgenommen.

Geeignete inerte organische Lösungsmittel sind z. B. gegebenenfalls chlorierte aliphatische oder aromatische Kohlenwassertoffe, wie Methylenchlorid, 1,1,2,2-Tetrachloräthan, Chloroform, Benzol, Toluol, Xylole, Chlorbenzol oder Dichlorbenzole ; aliphatische und cyclische Aether, wie Diäthyläther, Diisopropyläther, Di-n-butyläther, 1,2-Dimethoxyäthan, Tetrahydrofuran und Dioxan ; Alkylester von aliphatischen Monocarbonsäuren mit insgesamt 2-8 C-Atomen, wie Essigsäuremethyl-, -äthyl- und -n-butylester, Buttersäureäthyl, und -n-butylester ; N,N-Dialkylamide von aliphatischen Monocarbonsäuren mit 1-3 C-Atomen im Säureteil, wie N,N-Dimethylformamid und N,N-Dimethylacetamid ; cyclische Amide, wie N-Methylpyrrolidon ; Dialkylsulfoxide, wie Dimethylsulfoxid ; aliphatische Ketone, wie Aceton und Methyläthylketon ; Alkylnitrile mit 2-5 C-Atomen, wie Acetonitril und Propionitril ; Hexamethylphosphorsäuretriamid ; Alkanole mit bis zu 6 C-Atomen, wie Methanol, Aethanol, Propanol, Isopropanol, n-Butanol, Iso- und tert-Butanol. Bevorzugte Lösungsmittel sind 1,1,2,2-Tetrachloräthan und vor allem Tetrahydrofuran und N,N-Dimethylformamid.

Handelt es sich bei den Ausgangsverbindungen der Formel II um Imide, so kann die Umsetzung mit den Verbindungen der Formel III bzw. deren Salzen auch im Zweiphasengemisch unter Phasen-Transfer-Katalyse durchgeführt werden. Dabie wird die wässrige Phase zweckmässig mit z. B. $HCO_3^-$, $CO_3^{2-}$ oder $OH^-$ alkalisch gestellt (pH 7-14). Die organische Phase besteht aus einem mit Wasser nicht mischbaren inerten organischen Lösungsmittel, z. B. Dichlormethan, Chloroform, Toluol oder Chlorbenzol. Als Phasen-Transferkatalysatoren verwendet man mit Vorteil Tetraalkylammonium- oder -phosphoniumsalze, z. B. Benzyltriäthylammoniumchlorid, Cetyltrimethylammoniumchlorid oder Tetrabutylphosphoniumchlorid. Dabei liegen die Reaktionstemperaturen vorzugsweise zwischen 0 °C und 150 °C.

Verbindungen der Formel I, worin $Y_1$ und $Y_2$ Sauerstoff und Y —NH— oder —O— darstellen, können auch dadurch erhalten werden, dass man eine Verbindung der Formel IV

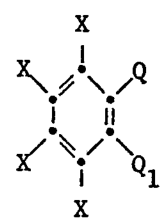

(IV)

worin X die unter Formel I angegebene Bedeutung hat und Q und $Q_1$ unabhängig voneinander —CN oder eine Carbonsäureestergruppe, bevorzugt eine niedere Carbonsäurealkylestergruppe, darstellen, auf die oben angegebene Weise direkt oder stufenweise mit einem Salz einer Verbindung der Formel III umsetzt. Die so erhaltenen Verbindungen der Formel V

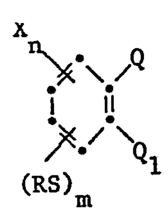

(V)

werden anschliessend in üblicher Weise unter sauren oder basischen Bedingungen hydrolysiert und cyclisiert. Die Cyclisierung erfolgt z. T. spontan, vor allem zu den Anhydriden, oder aber durch Erhitzen oder Zusatz von üblichen Dehydratisierungsmitteln.

Die nach den obigen Verfahren erhaltenen Anhydride der Formel I können gewünschtenfalls mit Aminen $H_2N—R'$ bzw. $H_2N—R''$ zu den entsprechenden Amidsäuren umgesetzt werden, die auf an sich bekannte Weise zu Imiden bzw. Isoimiden cyclisiert werden können. Die Cyclisierung zu Imiden der Formel I erfolgt im allgemeinen durch blosses Erhitzen oder unter Verwendung üblicher Dehydratisierungsmittel, wie Acetanhydrid. Die Cycliserung zu Isoimiden der Formel I kann z. B. nach den in den U.S. Patenschriften 2.995.577, 2.998.429, 3.035.065, 3.035.065 und 4.179.444 beschriebenen Verfahren vorgenommen werden. Geeignete Dehydratisierungsmittel zur Überführung in Isoimide der Formel I sind beispielsweise Keten, Carbodiimide, Trifluoracetanhydrid, Chloracetylchlorid oder Dichloracetylchlorid, gegebenenfalls im Gemisch mit tertiären Aminen. Isoimide der Formel I werden bevorzugt nach dieser Methode hergestellt.

Die allfällige Isomerisierung der Isoimide der Formel I zu entsprechenden Imiden kann ebenfalls in an sich bekannter Weise erfolgen, z. B. durch blosses Erhitzen oder nach den in den U.S. Patentschriften 2.980.694 und 4.132.751 beschriebenen Verfahren, beispielsweise durch Behandeln mit Alkalimetall- oder Ammoniumsalzen niederer Fettsäuren, wie Natriumacetat oder Triäthylammoniumacetat, oder Gemischen aus tertiären Aminen und Phenol.

Die Verbindungen der Formel I können als Sensibilisatoren für lichtvernetzbare Polymere der verschiedensten Art eingesetzt werden. Derartige Polymere werden z. B. zur Herstellung von Druckplatten für das Offsetdruckverfahren, zur Herstellung von Photooffset-Lacken, für die unkonventionelle Photographie, z. B. zur Herstellung von photographischen Bildern mittels Photopolymerisation oder Photovernetzung verwendet. Solche Polymere finden insbesondere Anwendung als sogenannte Photoresists zur Herstellung von gedruckten Schaltungen nach an sich bekannten Methoden. Dabei wird die mit der lichtempflindlichen Schicht versehene Seite der Leiterplatte durch ein das Leiterbild aufweisendes Dianegativ belichtet und dann entwickelt, worauf man die unbelichteten Stellen der Schicht durch Entwicklungsflüssigkeit herausholt.

Als Polymere können an sich beliebige Materialien verwendet werden, deren Lichtempfindlichkeit (Empfindlichkeit gegenüber aktinischen Strahlen) sich durch den Einsatz der erfindungsgemässen Sensibilisatoren erhöhen lässt. Ganz besonders eignen sich die Verbindungen der Formel I als Sensibilisatoren für Polymere der in der deutschen Offenlegungsschrift 2.626.769 beschriebenen Art, d. h. Polymere, die als lichtempfindliche Gruppen solche der Formel VI

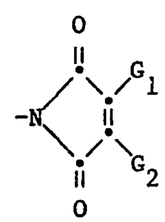

(VI)

aufweisen, worin $G_1$ und $G_2$ unabhängig voneinander Alkyl mit 1-4 C-Atomen, besonders Methyl, oder $G_1$ und $G_2$ zusammen die Ergänzung zu einem fünf- bis sechsgliedrigen carbocyclischen Ring bedeuten.

Die Verbindungen der Formel I können auf an sich bekannte Weise in die lichtvernetzbaren Polymeren eingearbeitet werden. Der Gehalt an Sensibilisator im Polymeren kann je nach Anwendungszweck und Anzahl der im Polymeren vorhandenen lichtvernetzbaren Gruppen stark variieren, liegt jedoch im allgemeinen zwischen etwa 0,1 und 20 % bezogen auf das Gewicht des Polymeren.

Schliesslich finden die Verbindungen der Formel I auch Anwendung als Photoinitiatoren. Die Erfindung betrifft daher auch die Verwendung der genannten Verbindungen zusammen mit Aminen als Initiatoren für die Photopolymerisation äthylenisch ungesättigter Verbindungen oder für die photochemische Vernetzung von Polyolefinen.

Die verwendeten organischen Amine können aliphatische, aromatische, araliphatische, cycloaliphatische oder heterocyclische Amine sein.

Sie können primäre, sekundäre oder tertiäre Amine sein. Beispiele hierfür sind : Butylamin, Dibutylamin, Tributylamin, cyclohexylamin, Benzyldimethylamin, Dicyclohexylamin, Triäthylamin, Phenyldi-äthanolamin, Piperidin, Piperazin, Morpholin, Pyridin, Chinolin, p-Dimethylaminobenzoesäureäthylester oder Michlers Keton (4,4'-Bis-dimethylamino-benzophenon).

Bevorzugt sind Gemische aus

A) einer Verbindung der Formel I, worin m, n, X, R, R' und R'' die im Vorangehenden angegebenen bevorzugten Bedeutungen haben, und

B) einem aliphatischen tertiären Amin, einem p-Dimethylaminobenzoesäurealkylester oder Michlers Keton.

Beispiele für aliphatische tertiäre Amine sind Trimethylamin, Triäthylamin, Tri-isopropyl-amin, Tributylamin, Dodecyldimethylamin, Octyl-dimethylamin, Triäthanolamin, Tris(hydroxypropyl)amin, N-Methyldiäthanolamin oder N-Butyl-diäthanolamin.

Besonders bevorzugt sind Gemische aus

A) einer Verbindung der Formel I, worin m, n, X, R, R' und R" die im Vorangehenden angegebenen bevorzugten Bedeutungen haben, und

B) Triäthanolamin oder einem $C_{1-4}$-Alkyldiäthanolamin.

Die genannten bevorzugten Gemische enthalten die Verbindungen der Formel I und die organischen Amine bevorzugt in einem Gewichtsverhältnis von 4 : 1 bis 1 : 4.

Photopolymerisierbare Verbindungen sind beispielsweise ungesättigte Monomere, wie Ester von Acryl- oder Methacrylsäure, z. B. Methyl-, Aethyl-, n- oder tert.-Butyl-, n.Octyl- der Hydroxyäthylacrylat, Methyl- oder Aethylmethacrylat, Aethylendiacrylat, Butandiodiacrylat, Hexandioldiacrylat, Neopentyldiacrylat, Trimethylolpropan-trisacrylat, Pentaerythrit-tetraacrylat oder Pentaerythrit-trisacrylat ; Acrylnitril, Methacrylnitril, Acrylamid, Methacrylamid, N-substituierte (Meth)-acrylamide ; Vinylester wie z. B. Vinylacetat, -propionat, -acrylat oder -succinat ; sonstige Vinylverbindungen, wie Vinyläther, Vinylketone, Vinylsulfone, Styrol, alkylstyrole, halogenstyrole, Divinylbenzol, N,N'-Divinylharnstoff, Vinylnaphthalin, N-Vinylpyrrolidon, Vinylchlorid oder Vinylidenchlorid ; Allylverbindungen, wie Diallylphthalat, Diallylmaleat, Triallylisocyanurat, Triallylphosphat oder Aethylenglykoldiallyläther und die Mischungen von solchen umgesättigten Monomeren. Besonders geeignet sind die erfindungsgemässen Gemische für die Photopolymerisation von Acrylsäureestern und deren Gemischen.

Weitere Beispiele sind ungesättigte Acrylharze. Hierzu zählen beispielsweise Umsetzungsprodukte von Polyepoxiden (Epoxidharzen) mit Acrylsäure oder Methacrylsäure oder Umsetzungsprodukte von Polyisocyanaten mit Hydroxyalkylacrylaten sowie die Umsetzungsprodukte von hydroxylgruppenhaltigen Polyestern oder Polyäthern mit Acryl- oder Methacrylsäure. Diese ungesättigten Acrylharze werden meist im Gemisch mit einem oder mehreren Acrylaten eines Mono-, Di- oder Polyalkohols, z. B. Aethyl-, Butyl-Benzyl-, 2-Aethylhexyl- oder 2-Hydroxypropylacrylat, Aethylenglykoldiacrylat, Propylenglykoldiacrylat, Butandioldiacrylat, Hexandioldiacrylat, Trimethylol-propan-trisacrylat oder Pentaerythrit-tetraacrylat, verwendet.

Gegenstand der Erfindung sind auch photopolymerisierbare Systeme, bestehend aus a) mindestens einer äthylenisch ungesättigten Verbindung, b) einem definitionsgemässen Gemisch aus A) und B) und gegebenenfalls c) sonstigen Zusatzstoffen, wie Inhibitoren, Stabilisatoren, UV-Absorbern, Füllstoffen, Pigmenten, Farbstoffen, Thixotropiemitteln und Verlaufshilfsmitteln, z. B. Silikonöl. Als Inhibitoren, welche vor allem während der Herstellung der Systeme durch Mischen der Komponenten vor einer vorzeitigen Polymerisation schützen sollen, werden beispielsweise Hydrochinon, Hydrochinon-derivate, p-Methoxyphenol oder ß-Naphthole verwendet. Als UV-Absorber können z. B. solche vom Benztriazol- oder Benzophenontyp eingesetzt werden. Als Füllstoffe kommen z. B. Kieselsäure, Talkum oder Gips in Betracht.

Bevorzugt sind derartige photopolymerisierbare Systeme in den Mengenverhältnissen 99,5-80 Gew.-% von a) und c) und 0,5-20 Gew.-% von b), wobei die Komponente A) vorzugsweise aus einer Verbindung der Formel I besteht, worin m, n, X, R, R' und R" die im Vorangehenden angegebenen bevorzugten Bedeutungen haben. Als Komponente a) verwendet man bevorzugt einen Acrylsäureester oder ein Gemisch mehrerer Acrylsäureester. Es können auch Kombinationen mit bekannten Photoinitiatoren, die durch Photofragmentierung Radikale bilden, wie z. B. Benzoinäther, Dialkoxyacetophenone oder Benzilketale, verwendet werden.

Grosse Bedeutung haben die erfindungsgemässen Initiatorgemische für die Photohärtung von Druckfarben und weiss pigmentierten Schichten, da die Trocknungszeit des Bindemittels ein massgeblicher Faktor für die Produktionsgeschwindigkeit graphischer Erzeugnisse ist und in der Grössenordnung von Bruchteilen von Sekunden liegen soll. Gut geeignet sind die erfindungsgemässen Initiatoren auch für photohärtbare Systeme zur Herstellung von Druckplatten. Ein weiteres Einsatzgebiet ist die UV-Härtung von Metallbeschichtungen, beispielsweise bei der Lackierung von Blechen für Tuben, Dosen oder Flaschenverschlüssen, sowie die UV-Härtung von Kunststoffbeschichtungen, beispielsweise von Fussböden- oder Wandbelägen auf PVC-Basis. Beispiele für die UV-Härtung von Papierbeschichtungen sind die farblose Lackierung von Etiketten, Schallplatten-Hüllen oder Buchumschlägen.

Die erfindungsgemässen Gemische können auch als Initiatoren zur photochemischen Vernetzung von Polyolefinen verwendet werden. Hierfür kommen z. B. Polypropylen, Polybuten, Polyisobutylen sowie Copolymerisate, wie z. B. Aethylen-Propylen-Copolymere in Frage, vorzugsweise jedoch Polyäthylen von niedriger, mittlerer oder hoher Dichte. Der Zusatz der Photoinitiatoren zu den photopolymerisierbaren Systemen geschieht im allgemeinen duch einfaches Einrühren, da die meisten dieser Systeme flüssig oder gut löslich sind. Meist kommt es zu einer Lösung der Initiatoren, wodurch deren gleichmässige Verteilung sowie die Transparenz der Polymerisate gewährleistet sind. Die Polymerisation erfolgt nach den bekannten Methoden der Photopolymerisation durch Bestrahlung mit Licht, das reich an kurzwelliger Strahlung ist. Als Lichtquellen sind z. B. Quecksilbermitteldruck-, -hochdruck- und -niederdruckstrahler, sowie superaktinische Leuchtstoffröhen geeignet, deren Emissionsmaxima im Bereich zwischen 250 und 450 nm liegen. Bei der photochemischen Vernetzung von Polyolefinen wird der Photoinitiator dem Polyolefin vor oder während der formgebenden Verarbeitung zugesetzt, beispielsweise durch pulverförmiges Vermischen oder durch Mischen mit dem plastifizierten Polyolefin. Die Vernetzung erfolgt durch Bestrahlung des geformten Gegenstandes in fester Form, beispielsweise in Form von Folien oder Fasern.

A) Herstellungsbeispiele

Beispiel 1

4,5-Bis-(Phenylthio)-3,6-dichlorphthalsäureanhydrid und 4-Phenylthio-3,5,6-trichlorphthalsäureanhydrid.

5,72 g (20 mMol) Tetrachlorphthalsäureanhydrid, 4,84 g (44 mMol), Thiophenol, 6,08 g (60 mMol) Triäthylamin und 50 ml 1,1,2,2-Tetrachloräthan werden 18 Std. bei 60 °C gerührt. Nach dem Eindampfen wird der Rückstand in 1N NaOH-Lösung gelöst, die Lösung wird angesäuert und mit Methylenchlorid/ Aceton extrahiert. Nach dem Umkristallisieren aus Toluol/Cyclohexan am Rückfluss erhält man 2,41 g (30 % d. Th.) 4,5-Bis-(Phenylthio)-3,6-dichlorphthalsäureanhydrid ; Smp. 204-7 °C.
Analyse für $C_{20}H_{10}Cl_2O_3S_2$ (Molgewicht 433,22) :

| | | | | | |
|---|---|---|---|---|---|
| berechnet | C 55,44 % | H 2,33 % | Cl 16,37 % | O 11,08 % | S 14,80 % |
| gefunden | C 55,05 % | H 2,35 % | Cl 16,29 % | O 11,16 % | S 14,83 %. |

Aus der Mutterlauge werden 0,91 g (13 % d. Th.) 4-Phenylthio-3,5,6-trichlorphthalsäureanhydrid erhalten ; Smp. 162-4 °C.
Analyse für $C_{14}H_5Cl_3O_3S$ (Molgewicht 359,61) :

| | | | | | |
|---|---|---|---|---|---|
| berechnet | C 46,76 % | H 1,40 % | Cl 29,58 % | O 13,53 % | S 8,92 % |
| gefunden | C 46,99 % | H 1,42 % | Cl 29,64 % | O 13,44 % | S 8,94 %. |

Beispiel 2

Tetrakis-(Phenylthio)-phthalsäureanhydrid.

30,88 g (108 mMol) Tetrachlorphthalsäureanhydrid, 48,79 g (443 mMol) Triophenol, 91,06 g (659 mMol) Kaliumcarbonat und 300 ml Tetrahydrofuran werden 3 Std. am Rückfluss gerührt. Man dampft ein und nimmt den Rückstand in Methylenchlorid/2N HCl-Lösung auf. Die organische Phase wird abgetrennt, über Natriumsulfat getrocknet und eingedampft. Nach dem Umkristallisiern aus Toluol/Cyclohexan erhält man 44,71 g (71 % d. Th.) Tetrakis-(Phenylthio)-phthalsäureanhydrid ; Smp. 141-2 °C.
Analyse für $C_{32}H_{20}O_3S_4$ (Molgewicht 580,75) :

| | | | |
|---|---|---|---|
| berechnet | C 66,18 % | H 3,47 % | S 22,08 % |
| gefunden | C 66,25 % | H 3,51 % | S 21,97 %. |

Beispiel 3

4,5-Bis-(Phenylthio)-3,6-dibromphthalsäureanhydrid.

2 g (4,31 mMol) Tetrabromphthalsäureanhydrid, 0,975 g (8,84 mMol) Thiophenol, 2,41 g (17,47 mMol) Kaliumcarbonat und 20 ml Tetrahydrofuran werden 2 Std. bei 0 °C und 2 Std. bei 25 °C gerührt. Das Gemisch wird mit 2 N CHl-Lösung angesäuert und mit Tetrahydrofuran/Toluol extrahiert. Die Extrakte werden über Natriumsulfat getrocknet und eingedampft. Nach dem Umkristallisieren aus Toluol/Cyclohexan erhält man 1,68 g (75 % d. Th.) 4,5-Bis-(Phenylthio)-3,6-dibromphthalsäureanhydrid ; Smp. 183-4 °C.
Analyse für $C_{20}H_{10}Br_2O_3S_2$ (Molgewicht 522,23) :

| | | | | |
|---|---|---|---|---|
| berechnet | C 46,00 % | H 1,93 % | Br 30,60 % | S 12,28 % |
| gefunden | C 46,25 % | H 2,03 % | Br 30,30 % | S 12,19 %. |

Beispiel 4

4-Phenylthio-3,5,6-trichlorphthalsäure-N-n-butylimid.

2 g (5,86 mMol) Tetrachlorphthalsäure-N-n-butylimid werden mit 2,43 g (17,59 mMol) Kaliumcarbonat in 15 ml Tetrahydrofuran bei 0 °C vorgelegt. Während 2 Std. wird eine Lösung von 680 mg (6,16 mMol) Thiophenolin 10 ml Tetrahydrofuran zugetropft. Das Gemisch wird 3 Std. bei 0 °C gerührt und während 6 Std. allmählich auf 25 °C erwärmt, dann mit 2 N HCl-Lösung angesäuert und mit Tetrahydrofuran/Toluol extrahiert. Nach dem Trocknen über Natriumsulfat wird die organische Phase eingedampft und der Rückstand wird mehrmals umkristallisiert. Man erhält 0,17 g (7 % d. Th.) 4-Phenylthio-3,5,6-trichlorphthalsäure-N-n-butylimid ; Smp. 159-63 °C.
Analyse für $C_{18}H_{14}Cl_3NO_2S$ (Molgewicht 414,73) :

| | | | | |
|---|---|---|---|---|
| berechnet | C 52,13 % | H 3,40 % | N 3,38 % | S 7,73 % |
| gefunden | C 52,36 % | H 3,44 % | N 3,39 % | S 8,25 %. |

Beispiel 5

4,5-Bis-(Phenylthio)-3,6-dichlorphthalsäure-N-n-butylimid.

20 g (58,65 mMol) Tetrachlorphthalsäure-N-n-butylimid, 33,64 g (243 mMol) Kaliumcarbonat, 13,57 g (123 mMol) Thiophenol und 150 ml Tetrahydrofuran werden 18 Std. bei 25 °C gerührt. Das Gemisch wird angesäuert und mit Methylenchlorid extrahiert. Die Extrakte werden getrocknet und eingedampft. Nach dem Umkristallisieren aus Toluol/Cyclohexan erhält man 23,88 g (83 % d. Th.) 4,5-Bis-(Phenylthio)-3,6-dichlorphthalsäure-N-n-butylimid ; Smp. 215-6 °C.

Analyse für $C_{24}H_{19}Cl_2NO_2S_2$ (Molgewicht 488,45) :

| | | | | | |
|---|---|---|---|---|---|
| berechnet | C 59,02 % | G 3,92 % | Cl 14,22 % | N 2,87 % | S 13,13 % |
| gefunden | C 59,01 % | G 3,98 % | Cl 14,40 % | N 3,22 % | S 13,27 %. |

Beispiel 6

Tetrakis-(Phenylthio)-phthalsäure-N-n-butylimid.

20 g (58,65 mMol) Tetrachlorphthalsäure-N-n-butylimid, 26,49 g (240,5 mMol) Thiophenol, 49,04 g (355 mMol) Kaliumcarbonat und 200 ml Essigsäureäthylester werden 18 Std. bei 25 °C gerührt und wie in den vorangehenden Beispielen beschrieben aufgearbeitet. Nach dem Umkristallisieren aus Toluol/Cyclohexan erhält man 35,99 g (97 % d. Th.) Tetrakis-(Phenylthio)-phthalsäure-N-n-butylimid ; Smp. 159-60 °C.

Analyse für $C_{36}H_{29}NO_2S_4$ (Molgewicht 635,87) :

| | | | | |
|---|---|---|---|---|
| berechnet | C 68,00 % | H 4,60 % | N 2,20 % | S 20,17 % |
| gefunden | C 67,99 % | H 4,74 % | N 2,25 % | S 19,99 %. |

Beispiel 7

4-(p-Toluylthio)-3,5,6-trichlorphthalsäure-N-2-hydroxyäthylimid.

2 g (6,08 mMol) Tetrachlorphthalsäure-n-2-hydroxyäthylimid, 0,79 g (6,38 mMol) p-Thiokresol, 2,52 g (18,24 mMol) Kaliumcarbonat und 20 ml Tetrahydrofuran werden 20 Std. bei 25 °C gerührt. Man säuert mit verdünnter HCl-Lösung an, extrahiert mit Methylenchlorid und trocknet über Natriumsulfat. Nach dem Umkristallisieren aus Toluol erhält man 1,41 g (56 % d. Th.) der genannten Verbindung ; Smp. 205-9 °C.

Analyse für $C_{17}H_{12}Cl_3NO_3S$ (Molgewicht 416,71) :

| | | | | | | |
|---|---|---|---|---|---|---|
| berechnet | C 49,00 % | H 2,90 % | Cl 25,52 % | N 3,36 % | O 11,52 % | S 7,69 % |
| gefunden | C 49,15 % | H 3,00 % | Cl 25,30 % | N 3,20 % | O 11,49 % | S 7,40 %. |

Beispiel 8

4,5-Bis(p-Toluylthio)-3,6-dichlorphthalsäure-N-2-hydroxyäthylimid.

20 g (60,8 mMol) Tetrachlorphthalsäure-N-2-hydroxyäthylimid, 15,86 g (127,67 mMol) p-Thiokresol, 34,03 g (246,22 mMol) Kaliumcarbonat und 200 ml Tetrahydrofuran werden 2,5 Tage bei 25 °C gerührt. Man säuert an und extrahiert mit Methylenchlorid. Nach dem Umkristallisieren aus Toluol erhält man 26,42 g (86 % d. Th.) der obigen Verbindung ; Smp. 211-2 °C.

Analyse für $C_{24}H_{21}Cl_2NO_3S_3$ (Molgewicht 506,46) :

| | | | | | |
|---|---|---|---|---|---|
| berechnet | C 56,92 % | H 4,18 % | Cl 14,00 % | N 2,76 % | S 12,66 % |
| gefunden | C 56,79 % | H 3,98 % | Cl 13,76 % | N 2,84 % | S 12,58 %. |

Beispiel 9

Tetrakis-(p-Toluylthio)-phthalsäure-N-2-hydroxyäthylimid.

2 g (6,08 mMol) Tetrachlorphthalsäure-N-2-hydroxyäthylimid, 3,1 g (24,93 mMol) p-Thiokresol, 5,08 g (36,78 mMol) Kaliumcarbonat und 20 ml N,N-Dimethylformamid werden 1 Std. bei 25 °C gerührt. Nach dem Eindampfen wird mit verdünnter HCl-Lösung angesäuert und mit Methylenchlorid extrahiert. Nach dem Umkristallisieren aus Toluol/Cyclohexan erhält man 3,21 g (78 % d. Th.) der oben genannten Verbindung ; Smp. 161-2 °C.

Analyse für $C_{38}H_{33}NO_3S_4$ (Molgewicht 679,93) :

| | | | | |
|---|---|---|---|---|
| berechnet | C 67,13 % | H 4,89 % | N 2,06 % | S 18,86 % |
| gefunden | C 67,29 % | H 4,97 % | N 2,24 % | S 18,55 %. |

## Beispiel 10

Tetrakis-(p-Nitrophenylthio)-phthalimid.

2 g (7,02 mMol) Tetrachlorphthalimid, 4,36 g (28,08 mMol) p-Nitrothiophenol, 5,82 g (42,12 mMol) Kaliumcarbonat und 30 ml Tetrahydrofuran werden 14 Std. bei 25 °C gerührt. Man säuert an, extrahiert mit Tetrahyrofuran/Toluol, trocknet die Extrakte und dampft ein. Der Rückstand wird mit Methylenchlorid gewaschen und aus Dioxan/Toluol umkristallisiert. Man erhält 3,02 g (57 % d. Th.) Tetrakis-(p-Nitrophenylthio)-phthalimid : Smp. 297-301 °C.

Analyse für $C_{32}H_{17}N_5O_{10}S_4$ (Molgewicht 759,76) :

| | | | | | |
|---|---|---|---|---|---|
| berechnet | C 50,59 % | H 2,26 % | N 9,22 % | O 21,06 % | S 16,88 % |
| gefunden | C 50,60 % | H 2,31 % | N 9,10 % | O 21,14 % | S 16,79 %. |

## Beispiel 11

Tetrakis-(p-Methoxyphenylthio)-phthalsäure-N-methylimid.

2 g (6,7 mMol) Tetrachlorphthalsäure-N-methylimid, 4,23 g (30,15 mMol) p-Methoxythiophenol, 6,25 g (45,2 mMol) Kaliumcarbonat und 20 ml Tetrahydrofuran werden 4 Std. bei 25 °C gerührt. Das Gemisch wird mit 2 N HCl-Lösung angesäuert und mit Methylenchlorid extrahiert. Die Extrakte werden über Natriumsulfat getrocknet und eingedampft. Nach dem Umkristallisieren aus Toluol/Cyclohexan erhält man 4,68 g (98 % d. Th.) der oben genannten Verbindung ; Smp. 168-70 °C.

Analyse für $C_{37}H_{31}NO_6S_4$ (Molgewicht 713,90) :

| | | | | |
|---|---|---|---|---|
| berechnet | C 62,25 % | H 4,38 % | N 1,96 % | S 17,96 % |
| gefunden | C 62,30 % | H 4,40 % | N 2,00 % | S 17,40 %. |

## Beispiel 12

4,5-Bis-(Phenylthio)-3,6-bis-(p-nitrophenylthio)-phthalsäure-N-n-butylimid.

2 g (4,09 mMol) 4,5-Bis-(Phenylthio)-3,6-dichlorphthalsäure-N-n-butylimid, 1,46 g (9,41 mMol) p-Nitrothiophenol, 2,55 g (18,45 mMol) Kaliumcarbonat und 20 ml Tetrahydrofuran werden 20 Std. 50 °C gerührt. Man kühlt ab, säuert an und extrahiert mit Methylenchlorid. Nach dem Umkristallisieren aus Tetrahydrofuran erhält man 1,9 g (78 % d. Th.) der genannten Verbindung ; Smp. 175-7 °C.

Analyse für $C_{36}H_{27}N_3O_6S_4$ (Molgewicht 725,87) :

| | | | |
|---|---|---|---|
| berechnet | C 59,57 % | H 3,75 % | N 5,79 % |
| gefunden | C 59,29 % | H 3,82 % | N 6,28 %. |

## Beispiel 13

Tetrakis-(n-Decylthio)-phthalsäure-N-2-hydroxyäthylimid.

2 g (6,08 mMol) Tetrachlorphthalsäure-N-2-hydroxyäthylimid, 5,30 g (30,4 mMol) n-Decanthiol, 6,30 g (45,6 mMol) Kaliumcarbonat und 20 ml N,N-Dimethylformamid werden 7 Std. bei 25 °C gerührt. Das Gemisch wird angesäuert und mit Methylenchlorid extrahiert. Nach dem Umkristallisieren aus n-Hexan bei 0 °C erhält man 3,75 g (70 % d. Th.) der obigen Verbindung ; Smp. 57-8 °C.

Analyse für $C_{50}H_{89}NO_3S_4$ (Molgewicht 880,51) :

| | | | | |
|---|---|---|---|---|
| berechnet | C 68,21 % | H 10,19 % | N 1,59 % | S 14,56 % |
| gefunden | C 68,15 % | H 10,09 % | N 1,70 % | S 14,48 %. |

## Beispiel 14

3,4,5-Tris-(p-Chlorphenylthio)-6-bromphthalimid und 4,5-Bis-(p-Chlorphenylthio)-3,6-dibromphthalimid.

2 g (4,46 mMol) Tetrabromphthalimid, 1,61 g (11,15 mMol) p-Chlorthiophenol, 2,31 g (16,73 mMol) Kaliumcarbonat und 20 ml Tetrahydrofuran werden 14 Std. bei 25 °C gerührt. Nach dem Ansäuern mit verdünnter HCl-Lösung wird mit Tetrahydrofuran/Toluol extrahiert, die Extrakte werden getrocknet und eingedampft. Durch Umkristallisieren aus Toluol/Cyclohexan werden 0,61 g (25 % d. Th.) 3,4,5-Tris-(p-Chlorphenylthio)-6-bromphthalimid erhalten ; Smp. 164-9 °C.

Analyse für $C_{26}H_{13}BrCl_3NO_2S_3$ (Molgewicht 653,84) :

berechnet C 47,76 % H 2,00 % Cl 16,27 % N 2,14 % O 4,89 % S 14,71 %
gefunden C 47,44 % H 2,26 % Cl 16,49 % N 2,42 % O 4,85 % S 14,70 %.

Aus der Mutterlauge werden 0,15 g (6 % d. Th.) 4,5-Bis-(p-Chlorphenylthio)-3,6-dibromphthalimid erhalten ; Smp. 310-5 °C.
Analyse für $C_{20}H_9Br_2Cl_2NO_2S_2$ (Molgewicht 558,07) :

berechnet C 40,71 % H 1,54 % Cl 12,01 % N 2,37 %
gefunden C 40,39 % H 1,77 % Cl 11,82 % N 2,69 %.

Beispiel 15

Tetrakis-(β-Hydroxyäthylthio)-phthalsäure-N-2-hydroxyäthylimid.

2 g (6,08 mMol) Tetrachlorphthalsäure-N-2-hydroxyäthylimid, 2,14 g (27,36 mMol) 2-Mercaptoäthanol, 5,46 g (39,52 mMol) Kaliumcarbonat und 20 ml N,N-Dimethylformamid werden 4 Std. bei 25 °C gerührt. Das Gemisch wird mit verdünnter HCl-Lösung angesäuert und mit Tetrahydrofuran/Toluol extrahiert. Nach dem Trocknen über Magnesiumsulfat werden die Extrakte eingedampft. Der Rückstand ergibt, aus Toluol/Cyclohexan umkristallisiert, 0,74 g (24 % d. Th.) der obigen Verbindung ; Smp. 152-7 °C.
Analyse für $C_{18}H_{25}NO_7S_2$ (Molgewicht 495,64) :

berechnet C 43,62 % H 5,08 % N 2,83 %
gefunden C 43,35 % H 5,00 % N 3,23 %.

Beispiel 16

3,6-Bis-(n-Decylthio)-4,5-bis-(p-toluylthio)-phthalsäure-N-2-hydroxyäthylimid.

2 g (3,95 mMol) 4,5-Bis-(p-Toluylthio)-3,6-dichlorphthalsäure-N-2-hydroxyäthylimid, 1,51 g (8,69 mMol) n-Decan-1-thiol, 2,29 g (16,58 mMol) Kaliumcarbonat und 20 ml N,N-Dimethylformamid werden 1 Std. bei 25 °C gerührt. Nach dem Einengen wird der Rückstand in Methylenchlorid/verdünnter HCl-Lösung aufgenommen. Die Extrakte werden über Natriumsulfat getrocknet und eingedampft. Nach dem Ausfällen aus n-Hexan erhält man 300 mg (10 % d. Th.) der obigen Verbindung (Substanz wachsartig).
Analyse für $C_{44}H_{61}NO_3S_4$ (Molgewicht 780,22) :

berechnet C 67,74 % H 7,88 % N 1,80 % S 16,44 %
gefunden C 67,61 % H 7,90 % N 1,82 % S 15,77 %.

Beispiel 17

4-(p-Methoxyphenylthio)-3,5,6-trichlorphthalsäure-N-methylimid.

41,41 g (138 mMol) Tetrachlorphthalsäure-N-methylimid, 19,42 g (138 mMol) p-Methoxythiophenol, 57,43 g Kaliumcarbonat und 410 ml Tetrahydrofuran werden 24 Std. bei 25 °C gerührt. Nach dem Eindampfen wird angesäuert und mit Methylenchlorid extrahiert. Nach dem Trocknen und Eindampfen kristallisiert man aus Toluol um. Man erhält 35,38 g (64 % d. Th.) des obigen Imids ; Smp. 182-6 °C.
Analyse für $C_{16}H_{10}NO_3S$ (Molgewicht 402,68) :

berechnet C 47,72 % H 2,50 % N 3,48 % S 7,96 % Cl 26,41 %
gefunden C 47,55 % H 2,55 % N 3,30 % S 8,26 % Cl 26,40 %.

Beispiel 18

Tetrakis-(o-Methoxyphenylthio)-phthalsäure-N-phenylimid.

2 g (3,71 mMol) Tetrabromphthalsäure-N-phenylimid, 2,34 g (16,7 mMol) o-Methoxythiophenol, 3,34 g (24,2 mMol) Kaliumcarbonat und 20 ml Tetrahydrofuran werden 24 Std. bei 25 °C gerührt. Nach dem Ansäuern wird mit Methylenchlorid extrahiert, getrocknet, eingedampft und aus Toluol umkristallisiert. Man erhält 2,75 g (96 % d. Th.) des obigen Imids ; Smp. 142-6 °C.
Analyse für $C_{42}H_{33}NO_6S_4$ (Molgewicht 775,97) :

berechnet C 65,01 % H 4,29 % N 1,81 % S 16,53 %
gefunden C 65,18 % H 4,33 % N 1,85 % S 16,30 %.

Beispiel 19

Tetrakis-(p-Nitrophenylthio)-phthalsäure-N-n-octylimid.

1 g (1,32 mMol) Tetrakis-(p-Nitrophenylthio)-phthalimid (hergestellt gemäss Beispiel 10), 0,57 g (2,97 mMol) 1-Bromoctan, 0,68 g (4,96 mMol) Kaliumcarbonat und 10 ml N,N-Dimethylformamid werden 5 Std. bei 25 °C gerührt. Das Gemisch wird eingeengt, und der Rückstand wird angesäuert und mit Methylenchlorid extrahiert. Nach dem Umkristallisieren aus Toluol/Cyclohexan erhält man 1,02 g (89 % d. Th.) des obigen Imids ; Smp. 202-4 °C.

Analyse für $C_{40}H_{33}N_5O_{10}S_4$ (Molgewicht 871,97) :

| | | | | |
|---|---|---|---|---|
| berechnet | C 55,10 % | H 3,82 % | N 8,03 % | S 14,71 % |
| gefunden | C 55,05 % | H 3,79 % | N 8,13 % | S 14,77 %. |

Beispiel 20

4,5-Bis-(Aethylthio)-3,6-dichlorphthalsäure-N-methylimid.

3 g (10,04 mMol) Tetrachlorphthalsäure-N-methylimid, 1,48 g (20,08 mMol) Aethanthiol, 5,55 g (40 mMol) Kaliumcarbonat und 30 ml N,N-Dimethylformalid werden 4 Std. bei 0 °C gerührt. Das Gemisch wird in Wasser aufgenommen und angesäuert, und das Reaktionsprodukt wird abfiltriert. Der Rückstand wird in Tetrahydrofuran/Toluol gelöst, die Lösung wird getrocknet und eingedampft. Nach dem Umkristallisieren aus Toluol/Cyclohexan erhält man 1,75 g (63 % d. Th.) des obigen Imids ; Smp. 154-5 °C.

Analyse für $C_{13}H_{13}Cl_2NO_2S_2$ (Molgewicht 350,28) :

| | | | | | |
|---|---|---|---|---|---|
| berechnet | C 44,58 % | H 3,74 % | Cl 20,24 % | N 4,00 % | S 18,30 % |
| gefunden | C 44,56 % | H 3,70 % | Cl 20,24 % | N 4,16 % | S 18,16 %. |

Beispiel 21

4,5-Bis-(n—$C_8F_{17}$—$CH_2CH_2$—thio)-3,6-dichlorphthalimid.

1 g (3,51 mMol) Tetrachlorphthalimid, 2,18 g (4,56 mMol) n—$C_8F_{17}CH_2CH_2$—SH, 1,89 g (13,7 mMol) Kaliumcarbonat und 15 ml N,N-Dimethylformamid werden 10 Std. bei 0 °C gerührt. Das Gemisch wird angesäuert und mit viel Wasser verrührt, der Niederschlag wird abfiltriert und in Tetrahydrofuran/Toluol gelöst. Die organische Phase wird mit $NaHCO_3$- und gesättigter NaCl-Lösung gewaschen, über Natriumsulfat getrocknet und eingedampft. Nach dem Umkristallisieren aus Toluol erhält man 2,53 g (99 % d. Th.) des obigen Imids ; Smp. 158-61 °C.

Analyse für $C_{28}H_9Cl_2F_{34}NO_2S_2$ (Molgewicht 1 172,34) :

| | | | | |
|---|---|---|---|---|
| berechnet | C 28,69 % | H 0,77 % | N 1,19 % | S 5,47 % |
| gefunden | C 28,68 % | H 0,81 % | N 1,33 % | S 5,54 %. |

Beispiel 22

4-(p-Toluylthio)-3,5,6-trichlorphthalsäure-N-methylimid.

2 g (6,7 mMol) Tetrachlorphthalsäure-N-methylimid, 950 mg (6,9 mMol) Kaliumcarbonat, 120 mg (0,33 mMol) Hexadecyltrimethylammoniumbromid, 20 ml Xylol und 0,86 ml Wasser werden bei 100 °C stark gerührt, und während 10 Minuten wird eine Lösung von 860 mg 6,9 mMol) p-Thiokresol in 20 ml Xylol zugetropft. Nach 30 Minuten wird abgekühlt, mit 2 N HCl-Lösung angesäuert und mit Methylenchlorid extrahiert. Nach dem Trocken über Natriumsulfat, dem Eindampfen und Umkristallisieren aus Toluol/Cyclohexan erhält man 310 mg (12 % d. Th.) des obigen Imids ; Smp. 209-16 °C.

Analyse für $C_{16}H_{10}Cl_3NO_2S$ (Molgewicht 386,68) :

| | | | | | | |
|---|---|---|---|---|---|---|
| berechnet | C 49,70 % | H 2,61 % | Cl 27,50 % | N 3,62 % | O 8,27 % | S 8,29 % |
| gefunden | C 49,76 % | H 2,06 % | Cl 27,35 % | N 3,89 % | O 8,38 % | S 8,19 %. |

Beispiel 23

4,5-Bis-(Phenylthio)-3,6-dichlorphthalsäure-N-phenylisoimid.

10,0 g (23 mMol) 4,5-Bis-(Phenylthio)-3,6-dichlorphthalsäureanhydrid und 100 ml Methylenchlorid werden vorgelegt. Unter Rühren wird eine Lösung von 2,147 g (23 mMol) Anilin in 25 ml Diäthyläther zugetropft. Nach 18 Std. Rühren und Kühlen wird das Reaktionsprodukt abfiltriert und getrocknet. Man

erhält 10,05 g (82 % d. Th.) 4,5-Bis-(Phenylthio)-3,6-dichlorphthalsäuremono-N-phenylamid ; Smp. 193-200 °C (Zers.).

Analyse für $C_{26}H_{17}NS_2O_3Cl_2$ (Molgewicht 526,45) :

| | | | | | |
|---|---|---|---|---|---|
| berechnet | C 59,32 % | H 3,25 % | N 2,66 % | S 12,18 % | Cl 13,47 % |
| gefunden | C 59,07 % | H 3,30 % | N 2,77 % | S 11,87 % | Cl 14,09 %. |

8,5 g (16 mMol) 4,5-Bis-(Phenylthio)-3,6-dichlorphthalsäure-mono-N-phenylamid und 40 ml $CH_2Cl_2$ werden vorgelegt. Dann werden 3,328 g (16 mMol) Dicyclohexylcarbodiimid in 20 ml $CH_2Cl_2$ zugetropft. Das Gemisch wird 18 Std. bei 25 °C gerührt, filtriert und die Mutterlauge wird eingedampft. Nach dem Umkristallisieren aus Methylenchlorid/n-Heptan erhält man 7,9 g (97 % d. Th.) 4,5-Bis-(Phenylthio)-3,6-dichlorphthalsäure-N-phenyl-isoimid : Smp. 158-160 °C.

Analyse für $C_{26}H_{15}Cl_2S_2NO_2$ (Molgewicht 508,44) :

| | | | | | | |
|---|---|---|---|---|---|---|
| berechnet | C 61,42 % | H 2,97 % | Cl 13,95 % | N 2,76 % | S 12,61 % | O 6,29 % |
| gefunden | C 61,25 % | H 3,04 % | Cl 14,23 % | N 3,08 % | S 12,40 % | O 6,36 %. |

Beispiel 24

4-(p-Toluylthio)-3,5,6-trichlorphthalsäure-N-methylimid.

2 g (6,69 mMol) Tetrachlorphthalsäure-N-methylimid werden bei 100 °C in 20 ml Xylol gelöst ; 0,86 ml Wasser, 0,95 g Kaliumcarbonat und 120 mg Hexadecyltrimethylammoniumbromid werden zugegeben und bei 100 °C eine Lösung von 0,86 g (6,89 mMol) p-Thiokresol in 1 ml Xylol zugetropft. Nach 30 Minuten wird das Gemisch abgekühlt, mit 2-n. HCl-Lösung angesäuert und mit Methylenchlorid extrahiert. Man trocknet mit Natriumsulfat, dampft die Lösung ein und kristallisiert den Rückstand aus Toluol/Cyclohexan um. Man erhält 0,69 g (27 %) der genannten Verbindung ; Smp. 212-216°.

Analyse für $C_{16}H_{10}Cl_3NO_2S$ (Molgewicht 386,68) :

| | | | | | | |
|---|---|---|---|---|---|---|
| berechnet | C 49,70 % | H 2,61 % | Cl 27,50 % | N 3,62 % | O 8,27 % | S 8,29 % |
| gefunden | C 49,76 % | H 2,60 % | Cl 27,35 % | N 3,89 % | O 8,38 % | S 8,19 % |

Beispiel 25

Tetrakis-(p-Toluylthio)-phthalsäureanhydrid.

20 g (70 mMol) Tetrachlorphthalsäureanhydrid, 37,36 g (301 mMol) p-Thiokresol, 60,91 g (441 mMol) Kaliumcarbonat und 200 ml Tetrahydrofuran werden während 2 Stunden am Rückfluss gerührt. Nach dem Eindampfen wird der Rückstand mit verdünnter HCl-Lösung angesäuert und mit Methylenchlorid extrahiert. Nach dem Umkristallisieren aus Cyclohexan erhält man 31,25 g (70 %) der genannten Verbindung ; Smp. 129-132°.

Analyse für $C_{36}H_{28}O_3S_4$ (Molgewicht 636,86) :

| | | | |
|---|---|---|---|
| berechnet | C 67,89 % | H 4,43 % | S 20,14 % |
| gefunden | C 68,18 % | H 4,39 % | S 19,87 % |

B) Anwendungsbeispiel

a) Herstellung des Polymeren

Es wird ein Polymeres mit folgender Struktur und Zusammensetzung hergestellt :

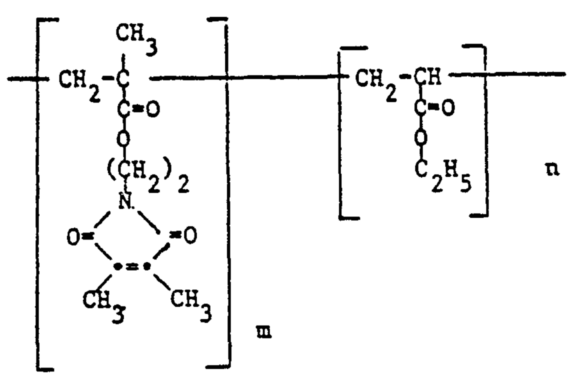

$$\frac{m}{m + n} = 0,8$$

$$\frac{n}{m + n} = 0,2$$

465,5 g (1,963 Mol) β-(Dimethylmaleinimidyl)-äthyl-methacrylat [hergestellt gemäss deutscher Offenlegungsschrift 2.626.769] werden zusammen mit 49,15 g (0,49 Mol) Acrylsäureäthylester unter Stickstoff in 960 ml 1-Acetoxy-2-äthoxyäthan gelöst. Bei 80 °C lässt man unter Stickstoffatmosphäre eine Lösung von 3,86 g Azoisobutyronitril in 25 ml 1-Acetoxy-2-äthoxyäthan zulaufen und polymerisiert dann währen 6 Stunden. Die noch heisse Lösung wird mit 2,57 g 2,6-Di-tert-butyl-p-kresol stabilisiert. Das durchschnittliche Molekulargewicht des so erhaltenen Polymeren (bestimmt durch Streulichtmessung in $CHCl_3$) beträgt $3 \times 10^6$ ; Grenzviskosität η grenz 0,8 dl/g ($CHCl_3$, 20 °C).

b) Erzeugung von Abbildungen

Zu 100 g der oben beschriebenen Polymerlösung in 1-Acetoxy-2-äthoxyäthan, verdünnt mit N,N-Dimethylformamid werden je $5 \times 10^{-3}$ Mol der in der Tabelle angegebenen Sensibilisatoren zugegeben, wobei sich die Menge (Konzentration) auf den Feststoffgehalt bezieht. Die Polymerlösungen mit dem gelösten Sensibilisator werden durch Aufschleudern (500 Umdrehungen/Minute während 1 Minute) so auf kupferkaschierte Epoxidplatten aufgetragen, dass nach dem Trocknen (15 Minuten bei 80 °C) eine 1-3 μ dicke Polymerschicht auf dem Kupfer gebildet wird. Die beschichteten Platten werden durch eine Negativ-Vorlage (Stufenkeil : Stouffer 21-Step-Sensitivity-Guide) mit einer 400 Watt Quecksilberhochdrucklampe im Abstand von 55 cm zum Vakuumtisch belichtet.

Vakuumtisch mit vorgeschaltetem Pyrex-Glasfilter von 8 mm Dicke.

Nach der Belichtung wird das Bild in einem 1,1,1-Trichloräthanbad während 2 Minuten entwickelt, wobei die unvernetzten Anteile herausgelöst werden. Das resultierende Reliefbild des abgebildeten Stufenkeils wird durch Aetzen der blanken Kupferteile mit einer 50 %igen $FeCl_3$-Lösung sichtbar gemacht. In der folgenden Tabelle bedeutet $S_{rel}$ die relative Empfindlichkeit. Sie ist ein Faktor, der angibt, um wieviel länger oder kürzer als 3 Minuten belichtet werden muss, um noch die Stufe 7 (optische Dichte des Stufenkeils = 1) abzubilden. Es gilt folgende Beziehung

$$S_{rel} = \frac{1}{\sqrt{2^{(7-X)}}} ,$$

wobei X die tatsächlich nach 3 Minuten Belichtung abgebildete Stufe bedeutet. Die Bestimmung von $S_{rel}$ basiert auf der von W.S. De Forest (« Photoresist », McGraw Hill Book Company, New York, 1975, Seiten 113 ff) beschriebenen Methode zur Ermittlung der Photoempfindlichkeit.

Tabelle

| Verbindung der Formel I | $\lambda_{max}$ | ε | $S_{rel}$ |
|---|---|---|---|
| Tetrakis-(p-Nitrophenylthio)-phthalsäure-N-n-octylimid | 332 | 50600 | 11,31 |
| 3,4,5-Tris-(p-Chlorphenylthio)-6-bromphthalimid | 345 | 8400 | 8,0 |
| 4,5-Bis-(Phenylthio)-3,6-dichlor-phthalsäure-N-n-butylimid | 376 | 4300 | 5,66 |
| 4-(p-Toluylthio)-3,5,6-trichlorphthalsäure-N-2-hydroxyäthylimid | 376 | 2760 | 4,00 |
| Tetrakis-(p-Nitrophenylthio)-phthalimid | 335 | 41800 | 4,00 |
| 4-(p-Methoxyphenylthio)-3,5,6-trichlor-phthalsäure-N-methylimid | nicht | bestimmt | 2,83 |
| 4-Phenylthio-3,5,6-trichlorphthalsäure-N-n-butylimid | 370 | 3000 | 2,83 |
| 4,5-Bis-(p-Toluylthio)-3,6-dichlor-phthalsäure-N-2-hydroxyäthylimid | 385 | 4600 | 2,83 |
| 4,5-Bis-(Phenylthio)-3,6-bis-(p-nitrophenylthio)-phthalsäure-N-n-butylimid | 351 | 25300 | 2,83 |
| Tetrakis-(Phenylthio)-phthalsäure-N-n-butylimid | 346 | 12600 | 1,40 |

(Fortsetzung)

| Verbindung der Formel I | $\lambda_{max}$ | $\varepsilon$ | $S_{rel}$ |
|---|---|---|---|
| Tetrakis-(o-Methoxyphenylthio)-phthalsäure-N-phenylimid | 348 | 11000 | 1,40 |
| 4,5-Bis-(Aethylthio)-3,6-dichlorphthalsäure-N-methylimid | 380 | 2100 | 1,00 |
| Tetrakis-(p-Toluylthio)-phthalsäure-N-2-hydroxyäthylimid | 353 | 12800 | 0,70 |
| Tetrakis-(p-Methoxyphenylthio)-phthalsäure-N-methylimid | 358 | 12300 | 0,34 |
| 3,6-Bis-(n-Decyl)-4,5-bis-(p-toluylthio)-phthalsäure-N-2-hydroxyäthylimid | 380 | 11200 | 0,18 |
| Tetrakis-(β-Hydroxyäthylthio)-phthalsäure-N-2-hydroxyäthylimid | 380 | nicht bestimmt | 0,13 |
| 4,5-Bis-(n-$C_8F_{17}$-$CH_2CH_2$-thio)-3,6-dichlorphthalimid | 340 | 2300 | 0,13 |
| Tetrakis-(n-Decylthio)-phthalsäure-N-2-hydroxyäthylimid | 310 | 11800 | 0,01 |
| 4,5-Bis-(Phenylthio)-3,6-dichlorphthalsäureanhydrid | 380 | 5100 | 4,00 |
| Tetrakis-(Phenylthio)-phthalsäureanhydrid | 355 | 13600 | 1,00 |
| 4,5-Bis-(Phenylthio)-3,6-dibromphthalsäureanhydrid | 385 | 5200 | 1,40 |
| 4,5-Bis-(Phenylthio)-3,6-dichlorphthalsäure-N-phenylisoimid | 377 | 10800 | 5,66 |

**Ansprüche**

1. Verbindungen der Formel I

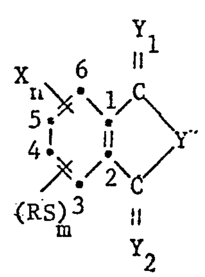

(I)

worin

m eine ganze Zahl von 1 bis 4 ist,

n 0, 1, 2 oder 3 darstellt und die Summe von m + n = 4 ist,

$Y_1$ und $Y_2$ Sauerstoff und Y—N—R' oder —O— oder eines von $Y_1$ und $Y_2$ Sauerstoff und das andere =N—R" und Y —O— bedeuten,

X ein Halogenatom,

die R (bei m > 1) unabhängig voneinander $C_{1\text{-}20}$-Alkyl, $C_{3\text{-}5}$-Alkenyl, $C_{3\text{-}5}$-Alkinyl, $C_{2\text{-}4}$-Monohydroxyalkyl, 2,3-Dihydroxy-1-propyl, —$OH_2COOH$, —$CH_2COO$—$C_{1\text{-}4}$-Alkyl, $C_{2\text{-}12}$-Halogenalkyl, Benzyl, $C_{5\text{-}12}$-Cycloalkyl, Phenyl, Halogenphenyl, Nitrophenyl, Alkyl- oder Alkoxyphenyl mit je 1-4 C-Atomen im Alkyl oder Alkoxy, Monohydroxyphenyl, Monoaminophenyl oder Monoacetylaminophenyl und

R' Wasserstoff, $C_{1\text{-}20}$-Alkyl, $C_{2\text{-}5}$-Alkenyl, $C_{3\text{-}5}$-Alkinyl, $C_{5\text{-}10}$-Cycloalkyl, Benzyl, Phenyl, Toluyl, $C_{2\text{-}8}$-Monohydroxyalkyl, gesättigter oder ungesättigter aliphatischer $C_{1\text{-}4}$-Carbonsäureester von $C_{2\text{-}8}$-Monohydroxyalkyl, $C_{1\text{-}12}$-Alkyläther von $C_{2\text{-}8}$-Monohydroxyalkyl, —N($C_{1\text{-}4}$-Alkyl)$_2$,

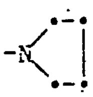 , 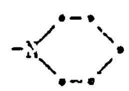 oder 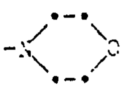

darstellen und

R'' dieselbe Bedeutung wie R' haben kann, aber nicht Wasserstoff darstellt, mit der Massgabe, dass die Gruppen —SR bei m = 1, 2 oder 3 in den Stellungen 4, 4,5 oder 3, 4,5 gebunden sind.

2. Verbindungen der Formel I nach Anspruch 1, worin für Y, $Y_1$, $Y_2$, m und n das im Anspruch 1 Angegebene gilt, X Chlor oder Brom, die R (bei m > 1) unabhängig voneinander geradkettiges $C_{1-12}$-Alkyl, 2-Hydroxyäthyl, Phenyl, p-Toluyl, 2-, 3- oder 4-Methoxyphenyl, 4-Chlorphenyl, 2,5-Dichlorphenyl, 4-Nitrophenyl, 4-Acetylaminophenyl, 2- oder 4-Aminophenyl, 4-Hydroxyphenyl oder $—CH_2CH_2C_pF_{2p+1}$ mit p = 6, 8 oder 10 und R' Wasserstoff, geradkettiges $C_{1-12}$-Alkyl, $C_{5-6}$-Cycloalkyl, Benzyl, Phenyl, Toluyl oder $C_{2-4}$-Monohydroxyalkyl darstellen und R'' dieselbe Bedeutung wie R' haben kann, jedoch nicht Wasserstoff darstellt.

3. Verbindungen der Formel I nach Anspruch 1, worin für Y, $Y_1$, $Y_2$, m und n das im Anspruch 1 Angegebene gilt, X Chlor oder Brom, die R unabhängig voneinander geradkettiges $C_{1-12}$-Alkyl, 2-Hydroxyäthyl, $—CH_2CH_2C_8F_{17}$, Phenyl, p-Toluyl, 2- oder 4-Methoxyphenyl, 4-Chlorphenyl, 2,5-Dichlorphenyl oder 4-Nitrophenyl, R' Wasserstoff, geradkettiges $C_{1-10}$-Alkyl, Phenyl, p-Toluyl oder 2-Hydroxyäthyl und R'' geradkettiges $C_{1-10}$-Alkyl, Phenyl, p-Toluyl oder 2-Hydroxyäthyl darstellen.

4. Verbindungen der Formel I nach Anspruch 1, worin für Y, $Y_1$, $Y_2$, m und n das im Anspruch 1 Angegebene gilt, X Chlor oder Brom, die R unabhängig voneinander geradkettiges $C_{1-12}$-Alkyl, 2-Hydroxyäthyl, $—CH_2CH_2C_8F_{17}$, Phenyl, p-Toluyl, 2- oder 4-Methoxyphenyl, 4-Chlorphenyl oder 4-Nitrophenyl, R' Wasserstoff, geradkettiges $C_{1-10}$-Alkyl, 2-Hydroxyäthyl oder Phenyl und R'' Phenyl darstellen.

5. Verbindungen der Formel I nach Anspruch 1, worin die R bei m = 2 je gleiche Gruppen darstellen.

6. Verbindungen der Formel I nach Anspruch 1, worin m die Zahl 4 darstellt und die R der in 3,6- und 4,5-Stellung gebundenen Gruppen —SR je paarweise dieselbe Bedeutung haben oder alle vier R die gleichen Gruppen darstellen.

7. Verfahren zur Herstellung von Verbindungen der Formel I nach Anspruch 1, dadurch gekennzeichnet, dass man eine Verbindung der Formel II

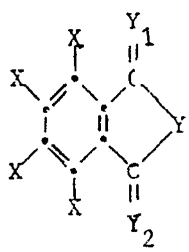

(II)

mit einer oder mehreren Verbindungen der Formel III

RSH (III)

oder Salzen von Verbindungen der Formel III umsetzt, dabei erhaltene Verbindungen der Formel I mit n = 1, 2 oder 3 gegebenenfalls mit weiterer Verbindung der Formel III bzw. weiterem Salz davon umsetzt und gegebenenfalls Verbindungen der Formel I, worin eines von $Y_1$ und $Y_2$ Sauerstoff und das andere =N—R'' und Y —O— bedeuten, zu den entsprechenden Imiden isomerisiert, wobei X, Y, $Y_1$, $Y_2$ und R die im Anspruch 1 angegebene Bedeutung haben.

8. Verfahren nach Anspruch 7, dadurch gekennzeichnet, dass man Anhydride der Formel I mit einem Amin $H_2N—R''$ zu den entsprechenden Amidsäuren umsetzt und die so erhaltenen Amidsäuren zu Isoimiden der Formel I cyclisiert.

9. Verwendung von Verbindungen der Formel I nach Anspruch 1 als Sensibilisatoren für lichtvernetzbare Polymere.

10. Verwendung von Verbindung der Formel I nach Anspruch 1 zusammen mit Aminen als Initiatoren für die Photopolymerisation äthylenisch ungesättigter Verbindungen oder für die photochemische Vernetzung von Polyolefinen.

**Claims**

1. A compound of the formula I

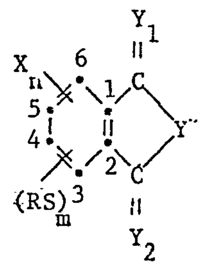

(I)

wherein

m is an integer from 1 to 4,

n is 0, 1, 2 or 3 and the sum of m + n is 4,

$Y_1$ and $Y_2$ are oxygen and Y is —N—R' or —O—, or one of $Y_1$ and $Y_2$ is oxygen and the other is =N—R'' and Y is —O—,

X is a halogen atom,

the radicals R (where m > 1) are each independently $C_1$-$C_{20}$alkyl, $C_{3-5}$alkenyl, $C_3$-$C_5$alkynyl, $C_{2-4}$ monohydroxyalkyl, 2,3-dihydroxyprop-1-yl, —$CH_2COOH$, —$CH_2COO$—$C_{1-4}$alkyl, $C_{2-12}$haloalkyl, benzyl, $C_{5-12}$cycloalkyl, phenyl, halophenyl, nitrophenyl, alkylphenyl or alkoxyphenyl, each containing 1-4 C atoms in the alkyl or alkoxy moiety, monohydroxyphenyl, monoaminophenyl or monoacetylaminophenyl, and

R' is hydrogen, $C_{1-20}$alkyl, $C_{2-5}$alkenyl, $C_{3-5}$alkynyl, $C_{5-10}$cycloalkyl, benzyl, phenyl, toluyl, $C_{2-8}$ monohydroxyalkyl, a saturated or unsaturated aliphatic $C_{1-4}$carboxylic acid ester of $C_{2-8}$monohydroxyalkyl, a $C_{1-12}$alkyl ether of $C_{2-8}$monohydroxyalkyl, —N($C_{1-4}$alkyl)$_2$,

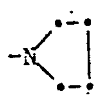 , 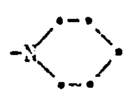 or 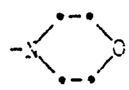

and

R'' can have the same meaning as R' but is not hydrogen, with the proviso that the —SR groups, where m is 1, 2 or 3, are bonded in the positions 4 or 4,5 or 3, 4 and 5.

2. A compound of the formula I according to claim 1, wherein Y, $Y_1$, $Y_2$, m and n are as defined in claim 1, X is chlorine or bromine, the radicals R (where m > 1) are earch independently straight-chain $C_{1-12}$-alkyl, 2-hydroxyethyl, phenyl, p-toluyl, 2-, 3- or 4-methoxyphenyl, 4-chlorophenyl, 2,5-dichlorophenyl, 4-nitrophenyl, 4-acetylaminophenyl, 2- or 4-aminophenyl, 4-hydroxyphenyl or —$CH_2CH_2C_pF_{2p+1}$ with p = 6, 8 or 10, and R' is hydrogen, straight-chain $C_{1-12}$alkyl, $C_{5-6}$cycloalkyln benzyl, phenyl, toluyl or $C_{2-4}$monohydroxyalkyl, and R'' can have the same meaning as R' but is not hydrogen.

3. A compound of the formula I according to claim 1, wherein Y, $Y_1$, $Y_2$, m and n are as defined in claim 1, X is chlorine or bromine, the radicals R are each indepently straight-chain $C_{1-12}$alkyl, 2-hydroxyethyl, —$CH_2CH_2C_8F_{17}$, phenyl, p-toluyl, 2- or 4-methoxyphenyl, 4-chlorophenyl, 2,5-dichlorophenyl or 4-nitrophenyl, R' is hydrogen, straight-chain $C_{1-10}$alkyl, phenyl, p-toluyl or 2-hydroxyethyl, and R'' is straight-chain $C_{1-10}$alkyl, phenyl, p-toluyl or 2-hydroxyethyl.

4. A compound of the formula I according to claim 1, wherein Y, $Y_1$, $Y_2$, m and n are as defined in claim 1, X is chlorine or bromine, the radicals R are each independently straight-chain $C_{1-12}$alkyl, 2-hydroxyethyl, —$CH_2CH_2C_8F_{17}$, phenyl, p-toluyl, 2- or 4-methoxyphenyl, 4-chlorophenyl or 4-nitrophenyl, R' is hydrogen, straight-chain $C_{1-10}$alkyl, 2-hydroxyethyl or phenyl, and R'' is phenyl.

5. A compound of the formula I according to claim 1, wherein the radicals R are each identical where m is 2.

6. A compound of the formula I according to claim 1, in which m is 4 and pairs of radicals R of the groups —SR bonded in the 3,6- and 4,5- positions each have the same meaning or all four radicals R are identical groups.

7. A process for the preparation of a compound of the formula I according to claim 1, which comprises reacting a compound of the formula II

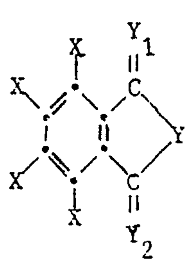

(II)

with one or more compounds of the formula III

RSH (III)

or salts of compounds of the formula III, if desired reacting the resulting compounds of the formula I in wich n = 1, 2 or 3 with a further compound of the formula III or a further salt thereof, and, if desired, isomerising compounds of the formula I in which one of $Y_1$ and $Y_2$ is oxygen and the other is =N—R″ and Y is —O—, to give the corresponding imides, and X, Y, $Y_1$, $Y_2$ and R are as defined in claim 1.

8. A process according to claim 7, wherein anhydrides of the formula I are reacted with an amine $H_2N$—R″ to give the corresponding amide acids, and the amide acids so obtained are cyclised to give isoimides of the formula I.

9. Use of a compound of the formula I according to claim 1 as a sensitiser for photocrosslinkable polymers.

10. Use of a compound of the formula I according to claim 1, together with amines, as initiator for the photopolymerisation of ethylenically unsaturated compounds or for the photochemical crosslinking of polyolefins.

**Revendications**

1. Composés répondant à la formule I

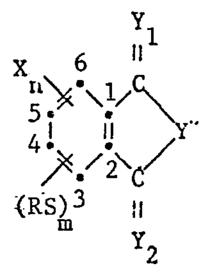

(I)

dans laquelle

m représente un nombre entier de 1 à 4,

n représente un nombre entier de 0 à 3, la somme (m + n) étant égale à 4,

ou bien $Y_1$ et $Y_2$ représentent chacun l'oxygène, auquel cas Y représente un radical $-\overset{|}{N}-R'$ ou —O—,

ou bien l'un des symboles $Y_1$ et $Y_2$ représente l'oxygène et l'autre un radical =N—R″, auquel cas Y représente —O—,

R ou les R (lorsque m est supérieur à 1) représentent chacun, indépendamment les uns des autres, un alkyle en $C_1$-$C_{20}$, un alcényle en $C_3$-$C_5$, un alcynyle en $C_3$-$C_5$, un monohydroxy-alkyle en $C_2$-$C_4$, un dihydroxy-2,3 propyle, —$CH_2COOH$, —$CH_2COO$-alkyl à alkyle en $C_1$-$C_4$, un halogéno-alkyle en $C_2$-$C_{12}$, un benzyle, un cyclo-alkyle en $C_5$-$C_{12}$, un phényle, un halogéno-phényle, un nitro-phényle, un alkyl- ou alcoxy-phényle contenant de 1 à 4 atomes de carbone dans sa partie alkyle ou alcoxy, un mono-hydroxy-phényle, un mono-aminophényle ou un mono-acétylamino-phényle,

R′ représente l'hydrogène, un alkyle en $C_1$-$C_{20}$, un alcényle en $C_2$-$C_5$, un alcynyle en $C_3$-$C_5$, un cycloalkyle en $C_5$-$C_{10}$, un benzyle, un phényle, un toluyle, un mono-hydroxy-alkyle en $C_2$-$C_8$, un radical ester d'acide carboxylique en $C_1$-$C_4$ aliphatique, saturé ou non, de mono-hydroxy-alkyle en $C_2$-$C_8$, un radical d'oxyde d'alkyle en $C_1$-$C_{12}$ et de mono-hydroxy-alkyle en $C_2$-$C_8$, —N(Alkyl en $C_1$-$C_4$)$_2$,

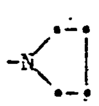 , 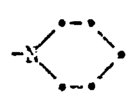 ou 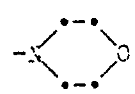

et

R″ a la même signification que R′ sans toutefois pouvoir représenter l'hydrogène, avec la condition que les radicaux —SR occupent, lorsque m est égal à 1, à 2 ou à 3, les positions 4, 4,5 ou 3, 4,5.

2. Composés de formule I selon la revendication 1 dans lesquels Y, $Y_1$, $Y_2$, m et n ont les significations données à la revendication 1 , X représente le chlore ou le brome, les R (lorsque m est supérieur à 1) représentent chacun, indépendamment les uns des autres, un alkyle linéaire en $C_1$-$C_{12}$, un hydroxy-2 éthyle, un phényle, un p-toluyle, un méthoxy-2, -3 ou -4 phényle, un chloro-4 phényle, un dichloro-2,5 phényle, un nitro-4 phényle, un acétylamino-4 phényle, un amino-2 ou -4 phényle, un hydroxy-4 phényle ou un radical —$CH_2CH_2C_pF_{2p+1}$ dans lequel p est égal à 6, à 8 ou à 10, R′ représente l'hydrogène, un alkyle linéaire en $C_1$-$C_{12}$, un cyclo-alkyle en $C_5$ ou $C_6$, un benzyle, un phényle, un toluyle ou un monohydroxy-alkyle en $C_2$-$C_4$, et R″ a la même signification que R′ mais ne peut pas représenter l'hydrogène.

3. Composés de formule I selon la revendication 1 dans lesquels Y, $Y_1$, $Y_2$, m et n ont les significations données à la revendication 1, X représente le chlore ou le brome, les R représentent chacun, indépendamment les uns des autres, un alkyle en $C_1$-$C_{12}$, un hydroxy-2 éthyle, $—CH_2CH_2C_8F_{17}$, un phényle, un p-toluyle, un méthoxy-2 ou -4 phényle, un chloro-4 phényle, un dichloro-2,5 phényle ou un nitro-4 phényle, R' représente l'hydrogène, un alkyle linéaire en $C_1$.$C_{10}$, un phényle, un p-toluyle ou un hydroxy-2 éthyle et R" représente un alkyle linéaire en $C_1$-$C_{10}$, un phényle, un p-toluyle ou un hydroxy-2 éthyle.

4. Composés de formule I selon la revendication 1 dans lesquels Y, $Y_1$, $Y_2$, m et n ont les significations données à la revendication 1, X représente le chlore ou le brome, les R représentent chacun, indépendamment les uns des autres, un alkyle linéaire en $C_1$-$C_{12}$, un hydroxy-2 éthyle, $—CH_2CH_2C_8F_{17}$, un phényle, un p-toluyle, un méthoxy-2 ou -4 phényle, un chloro-4 phényle ou un nitro-4 phényle, R' représente l'hydrogène, un alkyle linéaire en $C_1$-$C_{10}$, un hydroxy-2 éthyle ou un phényle, et R" représente un phényle.

5. Composés de formule I selon la revendication 1 dans lesquels les R, dans le cas où m est égal à 2, représentent des radicaux identiques.

6. Composés de formule I selon la revendication 1 dans lesquels m représente le nombre 4, les R occupant les positions 3 et 6 sont identiques à l'un à l'autre, les R occupant les positions 4 et 5 étant également identiques l'un à l'autre, et les quatre radicaux R pouvant également être identiques.

7. Procédé de préparation de composés de formule I selon la revendication 1, procédé caractérisé en ce qu'on fait réagir un composé de formule II

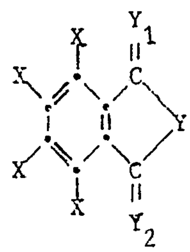

(II)

avec un ou plusieurs composés de formule III

RSH (III)

ou sels de composés de formule III, on fait éventuellement réagir les composés de formule I ainsi obtenus, si n est égal à 1, à 2 ou à 3, avec une quantité supplémentaire du composé de formule III ou d'un de ses sels, et on isomérise éventuellement des composés de formule I dans lesquels l'un des symboles $Y_1$ et $Y_2$ représente l'oxygène, l'autre représente un radical =N—R" et Y représente —O—, afin de les convertir en les imides correspondants (les symboles X, Y, $Y_1$, $Y_2$ et R ont les significations données à la revendication 1).

8. Procédé selon la revendication 7, caractérisé en ce qu'on fait réagir des anhydrides de formule I avec une amine $H_2N—R''$ de manière à obtenir les amides-acides correspondants, et on cyclise ces derniers en iso-imides de formule I.

9. Application de composés de formule I selon la revendication 1 comme sensibilisateurs pour des polymères photoréticulables.

10. Application de composés de formule I selon la revendication 1, associés à des amines, comme amorceurs pour la photopolymérisation de composés éthyléniques ou pour la réticulation photochimique de polyoléfines.